# EUROPEAN PATENT SPECIFICATION

(11) **EP 1 986 650 B1**
(45) Date of publication and mention of the grant of the patent: **13.06.2012**
(21) Application number: 07704144.0
(22) Date of filing: 25.01.2007
(51) Int. Cl.: A61K 9/20, A61K 31/485, A61K 9/22

(54) **TAMPER RESISTANT DOSAGE FORMS**
ORIGINALITÄTSGESICHERTE DOSIERUNGSFORMEN
FORMES DE DOSES INFALSIFIABLES

(30) Priority: 27.01.2006 EP 06001754
(43) Date of publication of application: 05.11.2008
(60) Divisional application: 10191037.0
(73) Proprietor: EURO-CELTIQUE S.A., 1653 Luxembourg (LU)
(72) Inventor: FLEISCHER, Wolfgang, 55218 Ingelheim (DE); LEUNER, Christian, 60439 Frankfurt (DE); SCHERER, Sabine, 65553 Limburg-Dietkirchen (DE)
(74) Representative: Bühler, Dirk
(86) International application number: PCT/EP2007/050751
(87) International publication number: WO 2007/085637

(56) References cited:
- EP-A- 1 604 666
- WO-A-99/32120
- WO-A-03/084504

## Description

### TECHNICAL FIELD

The present invention is directed to the prevention of the illicit use of opioid agonist dosage forms. The present invention is in particular directed to the prevention of the illicit use of oxycodone dosage forms.

### BACKGROUND

Pharmaceutical products are sometimes the subject of abuse. For example, a particular dose of opioid agonist may be more potent when administered parenterally as compared to the same dose administered orally. Some formulations can be tampered with to provide the opioid agonist contained therein for illicit use. Drug abusers sometimes try to achieve euphoric effects by manipulating drug formulations to quicken the onset. Controlled release opioid agonist formulations are sometimes crushed, or subject to extraction with solvents by drug abusers to provide the opioid contained therein for immediate release upon oral or parenteral administration.

The most rudimentary way of accomplishing this is by crushing the dosage form into a fine powder in an attempt to make the active ingredient more available. Oral abusers chew and/ or swallow the material, and nasal abusers crush the formulations for snorting.

In addition to the aforementioned "direct tampering" techniques, more determined abusers can also use various kinds of "kitchen chemistry" in an attempt to completely isolate the active ingredient from a formulation matrix. One method involves one-step extractions into commonly available media such as water or ethanol and mixtures thereof.

An effective amount of opioid antagonist can be used in opioid agonist dosage forms to induce tamper resistance. Opioid antagonists have the effect of antagonising the effect of opioid agonists. Therapeutically effective but tamper resistant oral dosage forms need to be effective when used correctly and ineffective enough , i.e. no sufficient effect of the opioid agonist, upon illicit use as for example crushing and extracting the dosage form to obtain an extract of the opioid agonist for parenteral administration. To prevent illicit use a dosage form comprising an opioid agonist and an opioid antagonist to induce temper resistance, the separation of the opioid agonist and opioid antagonist by extraction of the dosage form must be prevented.

Naloxone is an example of a known opioid antagonist useful to antagonize the effect of for example oxycodone. Oral administration of the Oxycodone/Naloxone combination results in release and absorption of both actives. Due to the high first pass metabolism naloxone has only a low oral bioavailability, while Oxycodone is active and systemically available. The dosage form is effective when used as intended, namely when used orally in form of e.g. a controlled release dosage form.

In a nasal or intravenous abuse situation there is no first pass metabolism. Both actives are systemic available and Naloxone antagonizes the drug action of Oxycodone. The combination therefore inhibits intravenous and nasal abuse.

To prevent illicit use of opioid agonist/opioid antagonist combination dosage forms separation of the opioid agonist from the opioid antagonist using common abuser extraction methods, i.e."kitchen chemistry", must be prevented.

The international patent application WO 03/084504 describes sustained release pharmaceutical preparations useful for the treatment of pain comprising oxycodone and naxolone, wherein oxycodone is not readily extracted from the mixture with naloxone, thus minimizing drug abuse possibilities.

### OBJECTS AND SUMMARY OF THE INVENTION

The present invention is directed to the prevention of the separation of the opioid agonist from the opioid antagonist from dosage forms comprising the opioid agonist and the opioid antagonist by simple extraction methods, commonly used by abusers.

Due to the illegal nature of these activities, there are no standardized methods for abusing pharmaceutical products. The experimental techniques used therein are designed to simulate commonly known methods of abuse.

The most rudimentary way to make the active ingredient available is by crushing the dosage form into a fine powder. Favoured methods for tampering oral dosage forms containing opioids are intravenous misuse and the simple one-step extraction.

It is an object of the invention to prevent separation of the opioid agonist oxycodone hydrochloride from the opioid antagonist naloxone hydrochloride from a dosage form comprising the opioid agonist and the opioid antagonist sufficient to make abuse unattractive.

It is an object of the invention to prevent the selective extraction of the opioid agonist form the dosage form comprising oxycodone hydrochloride and naloxone hydrochloride.

The present invention is directed to the use of an amount of naloxone hydrochloride in an amount at least sufficient to substantially antagonize a therapeutic amount of oxycodone hydrochloride when both, the opioid agonist and the opioid antagonist, are administered intravenous at the same time, in the form of a controlled release dosage form comprising a homogeneous controlled release matrix formulation comprising a hydrophobic material, comprising at least one hydrophobic polymer wherein the hydrophobic polymer is ethyl cellulose, and at least one fatty alcohol or fatty acid selected from C₁₂ to C₃₆ aliphatic alcohols or acids and said therapeutic amount of an opioid agonist and said sufficient amount of opioid antagonist in an amount ratio of 2:1.

The difference in the relative amounts of opioid agonist and opioid antagonist extracted by an extraction test, based on the total amounts present in the extracted dosage form before extraction, is useful to describe the separability of the opioid agonist from the opioid antagonist by extraction. The difference (Δ % points of the relative amounts extracted) should be sufficiently small to prevent euphoria as normally expected by the average abuser provided by the intravenous administration of the extract or there should be no difference or the relative amount of antagonist extract should be larger than the relative amount of agonist extracted.

According to one embodiment, the invention is directed to the use of an amount of an opioid antagonist in an amount at least sufficient to substantially antagonize a therapeutic amount of opioid agonist when both ,the opioid agonist and the opioid antagonist, are administered intravenous at the same time, in the form of a controlled release dosage form comprising a homogeneous controlled release matrix formulation comprising a hydrophobic material, comprising at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of a) crushing the formulation of one dosage form using a pill crusher or a tablet mortar, or using two spoons, wherein the crushing is performed at least 4 times using the spoons, b) extracting the crushed formulation of one dosage form on a spoon using 2 ml boiling tap water as extracting agent and a cigarette lighter as heating means for a time period that is necessary to boil the water, and c) filtering the solution using cotton, wherein the opioid antagonist is present in said extract in a weight percent amount, based on the total amount of opioid antagonist in the dosage form, that is more than 20 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

According to another embodiment the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of crushing the formulation of one dosage form using a pill crusher or a tablet mortar, or using two spoons, wherein the crushing is performed at least 4 times using the spoons, extracting said crushed formulation on a spoon using 2 ml boiling deionized water as extracting agent and a cigarette lighter as heating means for a time period that is necessary to boil the water, and filtering the solution using cotton, wherein the opioid antagonist is present in said extract in a weight percent amount, based on the total amount of opioid antagonist in the dosage form, that is more than 15 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

According to a further embodiment, the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of crushing the formulation of 10 dosage form using a pill crusher, extracting said crushed formulation in a glass vial using 100 ml of extraction solvent selected from the group of deionized water, hydrochloride acid (2N), acetic acid (2N), sodium hydroxide solution (0.1N, 0.5N, 1N or 2 N) and ethanol (40%), and shaking for at least 15 minutes at least at room temperature, wherein the opoid antagonist is present in said extract in a weight percent amount, based on the total amount of opioid antagonist in the dosage form, that is more than 10 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

According to a further embodiment, the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose and at least one fatty alcohol or fatty acids selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of: heating deionized water to 70°C, adding the intact formulation of one dosage form and stirring for 15 minutes, separating the extract, wherein the opioid antagonist is present in said extract in a weight percent amount, based on the total amount of opioid antagonist in the dosage form, that is more than 15 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

The term "sufficient to substantially antagonize a therapeutic amount of opioid agonist when both, the opioid agonist and the opioid antagonist, are administered intravenous at the same time" means that no euphoria is caused by said combined intravenous administration in an average abuser.

The term "controlled release matrix formulation" refers to the composition including the controlled release materials and the opioid.

The term "substantially homogenous controlled release matrix formulation" as used herein refers to a matrix formulation wherein the formulation compounds which form the matrix comprising the opioid agonist and the opioid antagonist form a uniform mixture of substances.

The term "controlled release dosage form" refers to the administration form comprising the "controlled release matrix formulation". The dosage form can be in the form of said formulation compressed into a tablet, optionally comprising further adjuvants, or in the form of a capsule comprising said formulation in the form of multi particulates, optionally comprising further adjuvants.

The opioids used according to the present invention may contain one or more asymmetric centers and may give rise to enantiomers, diastereomers, or other stereoisomeric forms. The present invention is also meant to encompass the use of all such possible forms as well as their racemic and resolved forms and mixtures thereof. When the compounds described herein contain olefinic double bonds or other centers of geometric asymmetry, it is intended to include both E and Z geometric isomers. All tautomers are intended to be encompassed by the present invention as well.

### BRIEF DESCRIPTION OF THE FIGURES

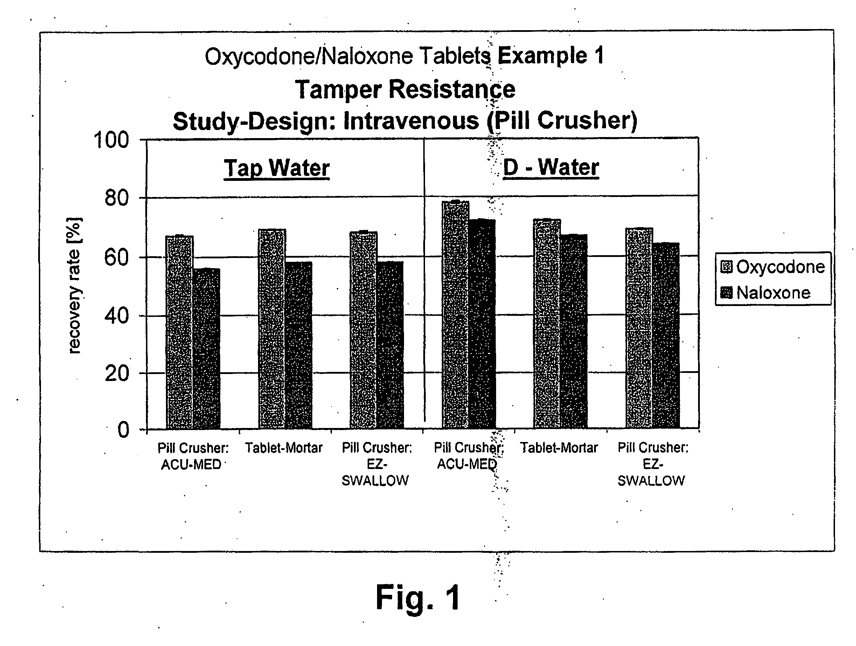

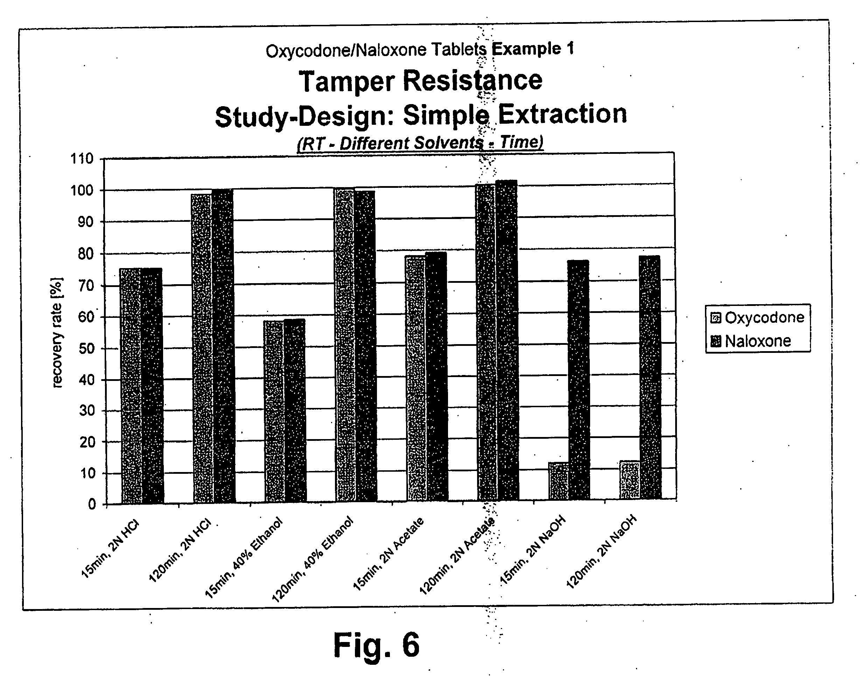

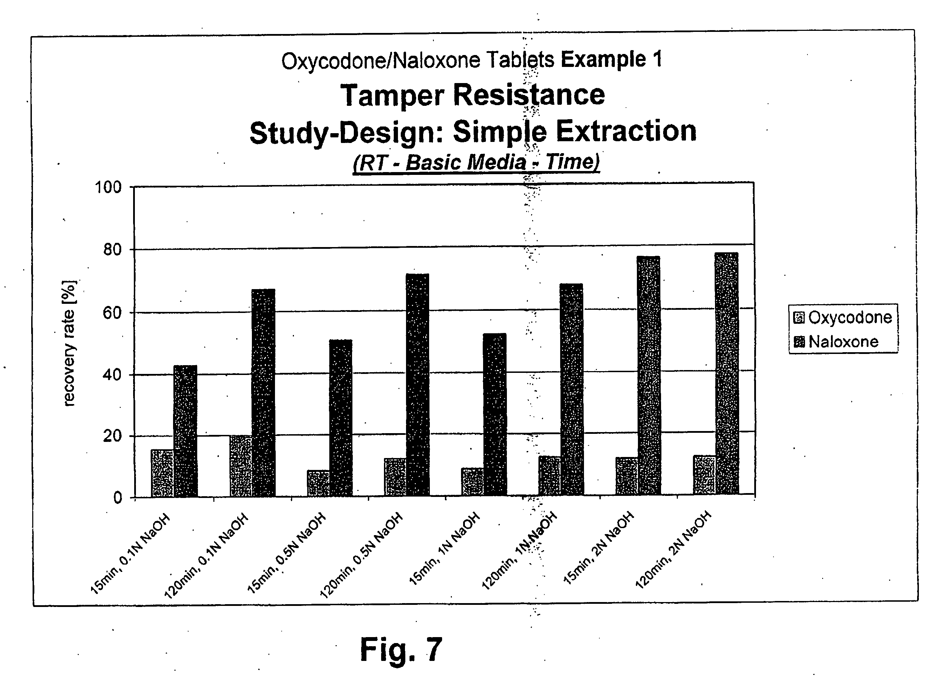

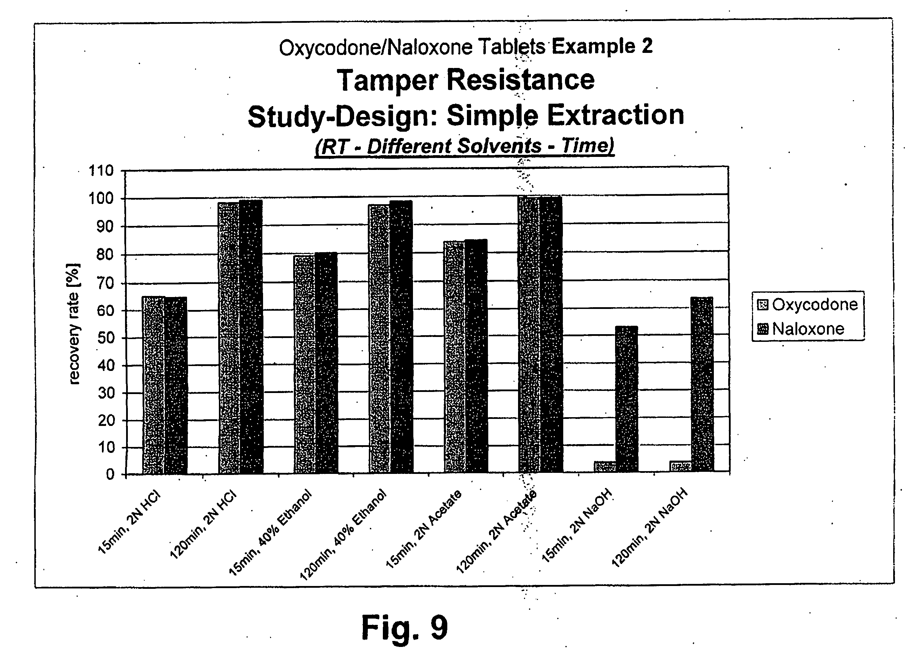

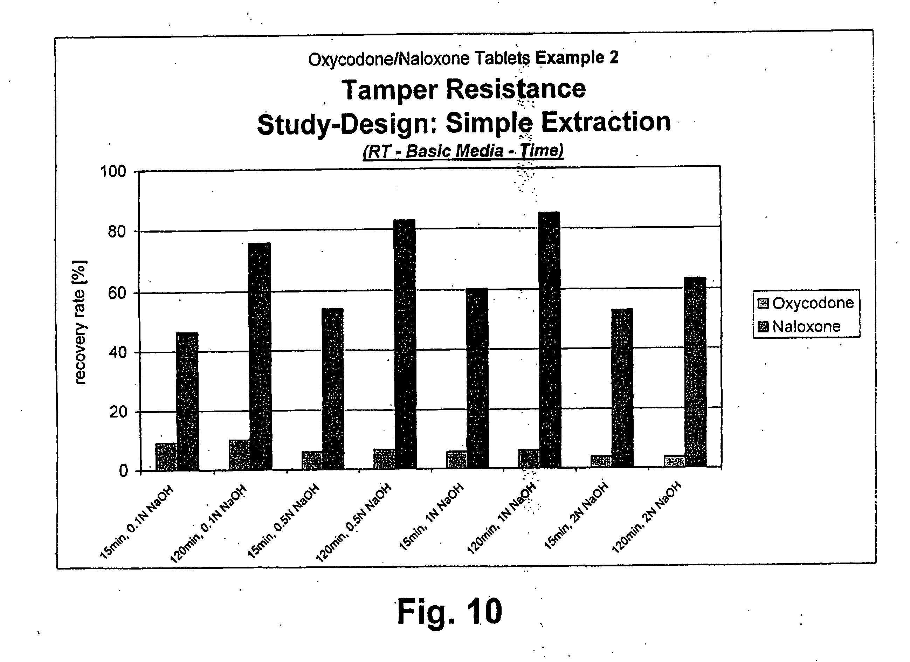

Figures 1 to 11 depict the extraction test results of Examples 1 and 2.

### DETAILED DESCRIPTION OF THE INVENTION

According to one embodiment the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of:
a) crushing the formulation of one dosage form using a pill crusher or a tablet mortar, or using two spoons, wherein the crushing is performed at least 4 times using the spoons,
b) extracting the crushed formulation of one dosage form on a spoon using 2 ml boiling tap water as extracting agent and a cigarette lighter as heating means for a time period that is necessary to boil the water, and
c) filtering the solution using cotton,
wherein the opioid antagonist is present in said extract in a weight percent amount, based on the total amount of opioid antagonist in the dosage form, that is more than 20 %-points, preferably more than 15 %-points, more preferably more than 12 %-points less than the weight percent amount of opioid agonist present in the extract, based on the total amount of opioid agonist in the dosage form, and wherein the opioid agonist is oxycodone hydrochloride and the opioid antoagonist is naloxone hydrochloride which are present in the dosage form in an amount ratio of 2:1.

In a further aspect the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of:
a) crushing the formulation of one dosage form using a pill crusher or a tablet mortar, or using two spoons, wherein the crushing is performed at least 4 times using the spoons
b) extracting said crushed formulation on a spoon using 2 ml boiling deionized water as extracting agent and a cigarette lighter as heating means for a time period that is necessary to boil the water, and
c) filtering the solution using cotton,
wherein the opioid antagonist is present in said extract in a weight percent amount, based on the total amount of opioid antagonist in the dosage form, that is more than 15 %-points, preferably more than 10 %-points, more preferably more than 7 %-points less than the weight percent amount of opioid agonist present in the extract, based on the total amount of opioid agonist in the dosage form, and wherein the opioid agonist is oxycodone hydrochloride and the opioid antoagonist is naloxone hydrochloride which are present in the dosage form in an amount ratio of 2:1.

In a further aspect the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of:
a) crushing the formulation of 10 dosage form using a pill crusher
b) extracting said crushed formulation in a glass vial using 100 ml of extraction solvent selected from the group of deionized water, hydrochloride acid (2N), acetic acid (2N), sodium hydroxide solution (0.1N, 0.5N, 1N or 2 N) and ethanol (40%), and shaking for at least 15 minutes at at least room temperature,
wherein the opioid antagonist is present in said extract in a weight percent amount, based on the total amount of opioid antagonist in the dosage form, that is more than 10 %-points, preferably more than 5 %-points and more preferably more than 3 % points less than the weight percent amount of opioid agonist present in the extract, based on the total amount of opioid agonist in the dosage form, and wherein the opioid agonist is oxycodone hydrochloride and the opioid antoagonist is naloxone hydrochloride which are present in the dosage form in an amount ratio of 2:1. Preferably, the formation of said extract is prevented, even wherein shaking is performed for 120 minutes. Preferably the formation of said extract is also prevented when deionized water is used as extraction solvent and during extraction the deionized water is heated to 50°C, preferably 75°C and most preferred 100°C for 5 minutes.

In a different aspect the invention is directed to the use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising a hydrophobic material, including at least one hydrophobic polymer wherein the hydrophobic polymer is ethyl cellulose, and at least one fatty alcohol or fatty acid selected from C₁₂ to C₃₆ aliphatic alcohols or acids, and said therapeutic amount of an opioid agonist and said sufficient amount of opioid antagonist, to prevent the formation of an extract of said controlled release matrix formulation comprising the opioid agonist by a one step extraction procedure comprising the steps of:
a) heating deionized water to 70°C
b) adding intact formulation of one dosage form and stirring for 15 minutes
c) separating the extract
wherein the opioid antagonist is present in said extract in a weight percent amount, based on the total amount of opioid antagonist in the dosage form, that is more than 15 %-points preferably more than 10 %-points less than the weight percent amount of opioid agonist present in the extract, based on the total amount of opioid agonist in the dosage form, and wherein the opioid agonist is oxycodone hydrochloride and the opioid antoagonist is naloxone hydrochloride which are present in the dosage form in an amount ratio of 2:1.

According to the invention the opioid agonist is oxycodone hydrochloride and the opioid antagoninst is naloxone hydrochloride used in an amount ratio of 2:1.

According to the invention the dosage form comprises a homogeneous controlled release matrix formulation comprising a hydrophobic material, including at least one hydrophobic polymer and at least one fatty alcohol or fatty acid, and said therapeutic amount of an opioid agonist and said sufficient amount of opioid antagonist.

The hydrophobic material is ethyl cellulose. Preferably the amount of ethyl cellulose is less than 20 % (by wt), preferably less than 15 % (by wt), most preferred less than 10 % (by wt) but more than 5 % (by wt) of the controlled release matrix formulation. The ethyl cellulose can be used in the form of particles or aqueous ethyl cellulose dispersions.

In case of ethyl cellulose particles, the ethyl cellulose has preferably a viscosity in the range of 3 to 110 cP, when measured in a 5 % solution at 25 °C in an Ubbelohde viscosimeter with a solvent of 80 % toluene and 20 % alcohol. Preferably, the viscosity is in the range of 18 to 110 cP and most preferred in the range of 41 - 49 cP. A suitable ethyl cellulose is provided by Dow Chemical Company under the trade name Ethocel ^{™} Standard 45.

In case of aqueous ethyl cellulose dispersions, a dispersion of ethyl cellulose 20 cP with dibutyl/sebacate, ammoniumhydroxide, oleic acid and colloidal anhydrous silica is preferred, which is available under the trade name Surlease ^{™} E-7-7050.

According to the present invention the hydrophobic polymer is used in combination with at least one second controlled release matrix material selected from C₁₂ to C₃₆ aliphatic alcohols and the corresponding aliphatic acids, preferably stearyl alcohol, cetyl alcohol and cetostearyl alcohol and the corresponding stearic and palmitic acids and mixtures thereof, wherein the amount of C₁₂ to C₃₆ aliphatic alcohol or aliphatic acid is preferably at least 5% more preferred at least 10% (by wt), more preferred at least 15 % (by wt) and most preferred 20 % to 25 % (by wt) of the controlled release matrix formulation.

The dosage form may comprise, besides the ethyl cellulose, and the C₁₂ to C₃₆ aliphatic alcohol or aliphatic acid, fillers and additional substances/adjuvants, such as granulating aids, lubricants, dyes, flowing agents and plasticizers.

Lactose, glucose or saccharose, starches and their hydrolysates, microcrystalline cellulose, cellatose, sugar alcohols such as sorbitol or mannitol, polysoluble calcium salts like calciumhydrogenphosphate, dicalcium- or tricalciumphosphat may be used as fillers.

Povidone may be used as granulating aid.

Highly-dispersed silica (Aerosil^{®}), talcum, corn starch, magnesium oxide and magnesium- or calcium stearate may preferably be used as flowing agents or lubricants.

Magnesium stearate and/or calcium stearate can be preferably be used as lubricants. Fats like hydrated castor oil can also preferably be used.

According to such certain embodiments, a formulation is especially preferred which comprises ethylcellulose, stearyl alcohol, magnesium stearate as lubricant, lactose as filler and providone as a granulating aid.

The production of the homogenous controlled release matrix formulation or preliminary stages thereof, which are in accordance with the invention, by extrusion technology is especially advantageous.

In one preferred embodiment, pharmaceutical formulations or preliminary stages thereof are produced by melt extrusion with co- or counter-rotating extruders comprising two screws. Another such preferred embodiment is the production by means of extrusion, with extruders comprising one or more screws. These extruders may also comprise kneading elements.

Extrusion is also a well-established production process in pharmaceutical technology and is well known to the person skilled in the art. The person skilled in the art is well aware that during the extrusion process, various parameters, such as the feeding rate, the screw speed, the heating temperature of the different extruder zones (if available), the water content, etc. may be varied in order to produce products of the desired characteristics.

The aforementioned parameters will depend on the specific type of extruder used. During extrusion the temperature of the heating zones, in which the components of the inventive formulation melt, may be between 40 to 120 °C, preferably between 50 to 100 °C, more preferably between 50 to 90 °C, even more preferably between 50 to 70 °C and most preferably between 50 to 65 °C, particularly if counter-rotating twin screw extruders (such as a Leistritz Micro 18 GGL) are used. The person skilled in the art is well aware that not every heating zone has to be heated. Particularly behind the feeder where the components are mixed, cooling at around 25 °C may be necessary. The screw speed may vary between 100 to 500 revolutions per minute (rpm), preferably between 100 to 250 rpm, more preferably between 100 to 200 rpm and most preferably around 150 rpm particularly if counter rotating twin screw extruders (such as a Leistritz Micro 18 GGL) are used. The geometry and the diameter of the nozzle may be selected as required. The diameter of the nozzle of commonly used extruders typically is between 1 to 10 mm, preferably between 2 to 8 mm and most preferably between 3 to 5 mm. The ratio of length versus diameter of the screw ofextruders that may be used for production of inventive preparations is typically around 40 : 1.

Generally, the temperatures of the heating zones have to be selected such that no temperatures develop that may destroy the pharmaceutically active compounds. The feeding rate and screw speed will be selected such that the pharmaceutically active compounds are released from the preparations produced by extrusion in a sustained, independent and invariant manner. If e.g. the feeding rate is increased, the screw speed may have to be increased correspondingly to ensure the same retardation.

The person skilled in the art knows that all the aforementioned parameters depend on the specific production conditions (extruder type, screw geometry, number of components etc.) and may have to be adapted such that the preparations produced by extrusion provide for the required release.

Preferably the C₁₂ to C₃₆ aliphatic alcohol or aliphatic acid melts and the ethylcellulose can be dissolved in said C₁₂ to C₃₆ aliphatic alcohol or aliphatic acid during the melt extrusion process to enhance homogeneity.

According to a preferred embodiment of the invention ethyl cellulose is used in an amount less than 10 % (by wt) but more than 5 % (by wt) of the matrix formulation and the C₁₂ to C₃₆ aliphatic alcohol is steary alcohol used in an amount of between 20% and 25% (by wt) and the opioid agonist is oxycodone hydrochloride and the opioid antagoninst is naloxone hydrochloride which are present in the dosage form in an amount ratio of 2:1 and the controlled release matrix formulation is prepared by a melt extrusion process.

According to the invention the resulting controlled release matrix formulation can be used in the form of multi particulates or the formulations can be formed into a tablet. The multi particulates or the tablet can be film coated. The film coat can provide further controlled release. In preferred embodiments the film coat does not provide further controlled release.

The invention is described by means of an oxycodone hydrochloride/naloxone hydrochloride with an amount ratio to 2:1, namely 10mg/5mg and 40mg/20mg.

Preparation of the Oxycodone/Naloxone dosage forms comprising 10mg/5mg and 20mg/40mg oxycodone hydrochloride and naloxone hydrochloride.

### EXAMPLE 1

Oxycodone/naloxone dosage form comprising 10 mg oxycodone hydrochloride and 5 mg naloxone hydrochloride

| Component | weight [mg/tabtet] |
|---|---|
| Oxycodone hydrochloride¹⁾ | 10.50 |
| corresponding to | |
| Oxycodone hydrochlorid anhydrous | 10.00 |

| Component | weight [mg/tablet] |
|---|---|
| naloxone hydrochloride dihydrate | 5.45 |
| corresponding to | |
| Naloxone hydrochlorid anhydrous | 5.00 |
| Povidone K30 | 5.00 |
| Ethyl cellulose 45 cp | 10.00 |
| Stearyl alcohol | 25.00 |
| Lactose monohydrate | 64.25 |
| Talc | 2.50 |
| Magnesium-Stearate | 1.25 |
| film coating opadry II HP white - 85F18422° | 3.72 |

| | |
|---|---|
| 1) calculated based on expected moisture content ° qualitative composition: see Table 1 | |

### EXAMPLE 2

Oxycodone/naloxone dosage form comprising 40 mg oxycodone hydrochloride and 20 mg naloxone hydrochloride

| Component | weight [mg/tablet] |
|---|---|
| Oxycodone hydrochloride¹⁾ | 42.00 |
| corresponding to | |
| Oxycodone hydrochlorid anhydrous | 40.00 |
| naloxone hydrochloride dihydrate | 21.80 |
| corresponding to | |
| Naloxone hydrochlorid anhydrous | 20.00 |

| Component | weight [mg/tablet] |
|---|---|
| Povidone K30 | 14.50 |
| Ethyl cellulose 45 cp | 24.00 |
| Stearyl alcohol | 59.00 |
| Lactose monohydrate | 109.00 |
| Talc | 5.00 |
| Magnesium-Stearate | 2.5 |
| film coating opadry II HP yellow 85F32109° | 8.33 |

| | |
|---|---|
| 2) calculated based on expected moisture content ° qualitative composition: see Table 1 | |

**TABLE 1:**

| Qualitative composition of the film coat | | | | |
|---|---|---|---|---|
| **Opadry II HP** | **white 85F18422** | **pink 85F24151** | **yellow 85F32109** | **Reference to Standard** |
| Polyvinylalcohol part. hydrolized | + | + | + | Ph. Eur. * |
| Titanium dioxide (E 171) | + | + | + | Ph. Eur.* |
| Macrogol 3350 | + | + | + | Ph. Eur. * |
| Talcum | + | + | + | Ph. Eur. * |
| Iron oxide red (E 172) | | + | | NF* /EC Directive |
| Iron oxide yellows (E 172) | | | + | NF* /EC Directive |
| | | | | * current Edition |

The above described dosage forms were prepared by melt extrusion.

Oxycodone hydrochloride and naloxone hydrochloride are blended with povidone, ethylcellulose, stearyl alcohol and lactose, the blend is screened to remove agglomerates and further blended. The blend is melt extruded utilizing a heated twin screw extruder, to form strands which are milled to produce granules. The granules are blended with talc and magnesium stearate, compressed into capsule shaped tablets, which are then film coated.

### TAMPER TESTS

### Used Materials

| | |
|---|---|
| 2ml Syringes | Injection needles |
| DB Plastipak™ | (0,90x40mm) |
| Batch 0502018 | 100 Sterican® |
| | Batch 98K2982510 |
| | B/Braun Melsungen/Germany |
| Cotton | |
| Lohmann Rauscher | |
| Batch 1055314 | |
| Rengsdorf, Germany | |
| | |
| ACU-Med Pill Crusher | EZ-SWALLOW™ Pill Crusher |
| Health Enterprises, Inc. | American Medical Industries |
| North Attleboro, MA 02760, USA | Dell Papids, USA |
| | |
| Tablet Mortar | |
| Medi-Globe® Vertriebs GmbH | |
| Eppstein, Germany | |

### Extraction Tests:

**1) (Intravenous)** The methods for evaluating the intravenous technique involved crushing a tablet of Example 1 or 2, followed by extraction into a small quantity of water. The resultant solution was then drawn into an insulin syringe. Crushing the tablets was accomplished by using different pill crushers and stainless steel tablespoons.

Using a heat extraction procedure the opioids are extracted from the crushed material. The procedure required 2ml water, tap water or deionized water (D-water), a cigarette lighter for heating the solution on the spoon, cotton to filter the solution, and insulin syringes to transfer the filtrate to a flask for analysis. Each experiment was repeated three times.

The quantity of oxycodone and naloxone extracted from the material was evaluated using an assay HPLC method with UV detection at 230nm wavelength. Percent recovery was calculated on the basis of the total amount of oxycodone and naloxone in the tablet that was determined at the beginning of the tests.

**2) (simple extraction)** To simulate tampering the product by simple extraction, the dosage form was crushed (10 tablets or Example 1 or 2/experiment) with a pill crusher, combined with 100ml of an extraction solvent (D-water, acidic, basic and 40% ethanol media), heated to a specified temperature, shaken for 15 minutes and 120 minutes and analysed for extractability. Each experiment was repeated 3 times.

The quantity of oxycodone and naloxone extracted from the material was evaluated using an assay HPLC method with UV detection at 230nm wavelength. Percent recovery was calculated on the basis of the total amount of oxycodone and naloxone that was determined at the beginning of the tests.

**3) (additional test)** To simulate the effect of swallowing the intact dosage form with a hot, non-alcoholic drink, deionized water (D-water) was heated to 70°C, the intact tablet of Example 1 or 2 was added and stirred for 15min. After cooling to room temperature, the murky solution was transferred to a flask and measured for its pH. Each experiment was repeated 3 times.
The quantity of oxycodone and naloxone extracted from the material (murky solution) was evaluated using an assay HPLC method with UV detection at 230 nm wavelength. Percent recovery was calculated on the basis of the total amount of oxycodone and naloxone that was determined at the beginning of the tests.

The details of the test procedures are summarized in Table 2 below.

**TABLE 2**

| **Tampering Technique** | **Equipment Required** | **Knowledge Required** | **Dosage Form Treatment** | **Extraction Time** | **Extraction Solvent(s)** | **Extraction Temperature (°C)** |
|---|---|---|---|---|---|---|
| **1) Intravenous** | Spoons. Pill Crusher. Syringe, Lighter, Cotton | Simple | Crushed | Time required to boil | Water | 100 (boiling) |
| **2) Simple EXtraction** | Pill Crusher Glass Vial for Shaking, Water Bath, Thermometer | Slightly More Advanced | Crushed | 15 minutes, 2 hours | Water 40% ethanol, HCl 2N, CH₃COOH 2N, NaOH 0.1N, 0.5N. 1N, 2N | RT 50.75, 100 |
| | | | | | | RT |
| | | | | | | RT |
| | | | | | | RT |
| | | | | | | RT |
| **3) Additional Test** | Glass Vial, Water Bath, Thermometer | Simple | Intact | 15 minutes | Water | 70 |

The test results are as follows.

### Test results "intravenous" using Example 1

| **Crushing Method** | **Water** | **% recovery Oxycodone*** | **% recovery Naloxone*** | **Δ% points** |
|---|---|---|---|---|
| Pill Crusher ACU-MED | Tap Water | 67 | 56 | 11 |
| Tablet-Mortar | Tap Water | 69 | 58 | 11 |
| Pill Crusher EZ-SWALLOW | Tap Water | 68 | 58 | 10 |
| Pill Crusher ACU-MED | D-Water | 78 | 72 | 6 |
| Tablet-Mortar | D-Water | 72 | 67 | 5 |
| Pill Crusher EZ-SWALLOW | D-Water | 69 | 64 | 5 |
| Two Spoons *(Crushed 4 times)* | Tap Water | 75 | 63 | 12 |
| Two Spoons *(Crushed 8 times)* | Tap Water | 72 | 60 | 12 |
| Two Spoons *(Crushed 4 times)* | D-Water | 80 | 74 | 6 |
| Two Spoons *(Crushed 8 times)* | D-Water | 82 | 75 | 7 |

| | | | | |
|---|---|---|---|---|
| • *Average of 3 replicates* | | | | |

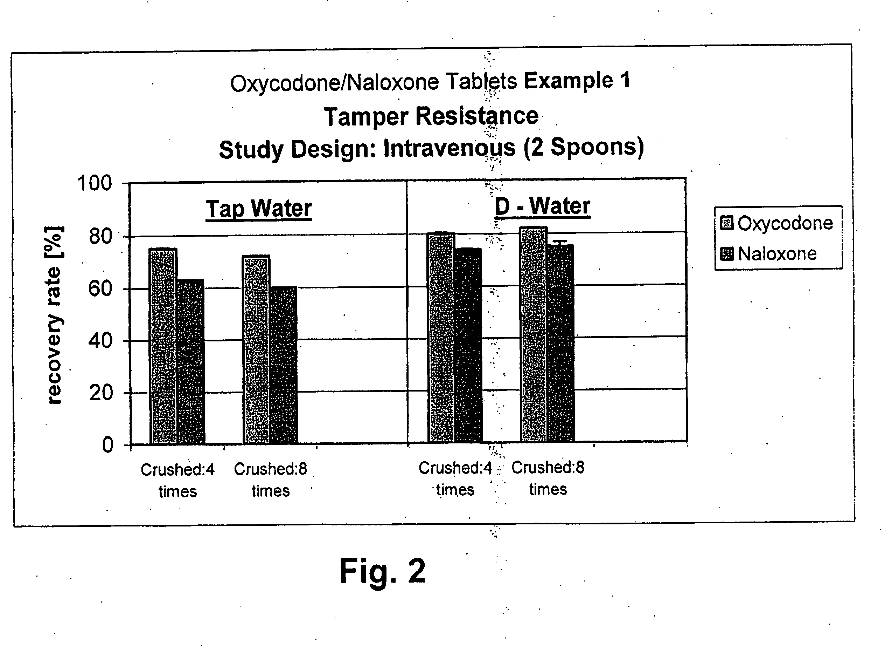

The results are also presented in Figures 1 and 2.

### Test results "intravenous" using Example 2

| **Crushing Method** | **Water** | **% recovery Oxycodone*** | **% recovery Naloxone*** | **A % points** |
|---|---|---|---|---|
| Pill Crusher ACU-MED | Tap Water | 73 | 66 | 7 |
| Tablet-Mortar | Tap Water | 67 | 61 | 6 |

| **Crushing Method** | **Water** | **% recovery Oxycodone*** | **% recovery Naloxone*** | **Δ % points** |
|---|---|---|---|---|
| Pill Crusher EZ-SWALLOW | Tap Water | 66 | 59 | 7 |
| Pill Crusher ACU-MED | D-Water | 76 | 72 | 4 |
| Tablet-Mortar | D-Water | 68 | 65 | 3 |
| Pill Crusher EZ-SWALLOW | D-Water | 70 | 67 | 3 |
| Two Spoons *(Crushed 4 limes)* | Tap Water | 77 | 69 | 8 |
| Two Spoons *(Crushed 8 times)* | Tap Water | 74 | 66 | 8 |
| Two Spoons *(Crushed 4 times)* | D-Water | 76 | 69 | 7 |
| Two Spoons *(Crushed 8 times)* | D-Water | 79 | 76 | 3 |

| | | | | |
|---|---|---|---|---|
| ** Average of 3 replicates* | | | | |

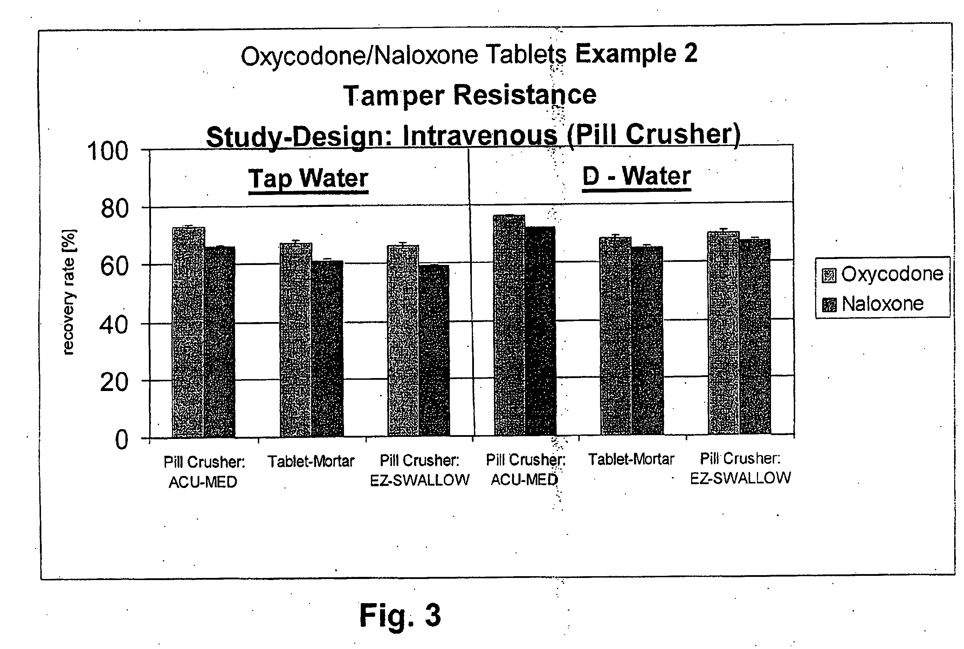

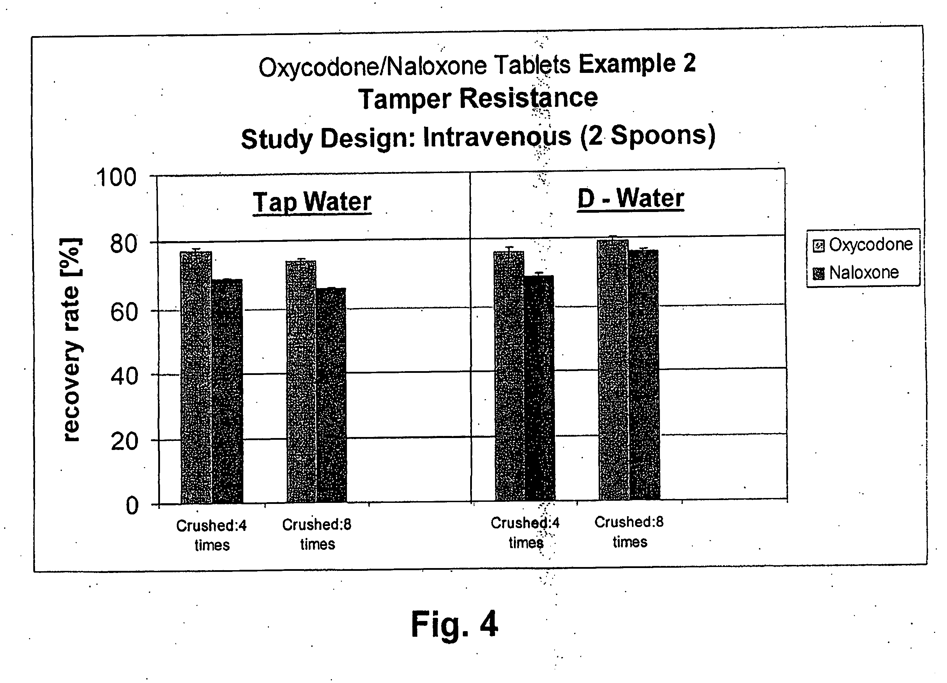

The results are also presented in Figures 3 and 4.

### Test results "simple extraction" using Example 1

| **Solvent** | **Temperature Time** | **Stirring/Time** | **PH (Test-solution)** | **% recovery Oxycodone*** | **% recovery Naloxone*** | **Δ% points** |
|---|---|---|---|---|---|---|
| D-Water | RT | 15min | 6.9 | 68 | 69 | 1 |
| D-Water | RT | 120min | 6.9 | 98 | 99 | 1 |
| D-Water | 50°C/5min | 15mm | 7.0 | 84 | 84 | 0 |
| D-Water | 50°C/5mm | 120min | 7.0 | 99 | 100 | 1 |
| D-Water | 75°C/5mm | 15min | 7.2 | 98 | 97 | 1 |
| D-Water | 75°C/5min | 120min | 7.0 | 98 | 98 | 0 |
| D-Water | 100°C/5min | 15min | 7.2 | 98 | 95 | 3 |

| **Solvent** | **Temperature/ Time** | **Stirring/Time** | **PH (Test-solution)** | **% recovery Oxycodone*** | **% recovery Naloxone*** | **Δ% points** |
|---|---|---|---|---|---|---|
| D-Water | 100°C/5min | 120min | 7.2 | 99 | 96 | 3 |
| HCl 2N | RT | 15min | not measurable | 75 | 75 | 0 |
| HCl 2N | RT | 120min | not measurable | 98 | 100 | 2 |
| Ethanol 40% | RT | 15min | 6.6 | 58 | 58 | 0 |
| Ethanol 40% | RT | 120min | 6.6 | 100 | 99 | 1 |
| CH₃COOH 2N | RT | 15min | 2.1 | 78 | 79 | 1 |
| CH₃COOH 2N | RT | 120min | 2.1 | 100 | 102 | 2 |
| NaOH 2N | RT | 15mm | 13.8 | 12 | 76 | 64 |
| NaOH 2N | RT | 120min | 13.8 | 12 | 77 | 65 |
| NaOH 1N | RT | 15 min | 13.7. | 9 | 52 | 53 |
| NaOH 1N | RT | 120min | 13.7 | 12 | 68 | 56 |
| NaOH 0.5N | RT | 15min | 13.5 | 8 | 51 | 43 |
| NaOH 0.5N | RT | 120min | 13.5 | 12 | 71 | 59 |
| NaOH 0.1N | RT | 15min | 13.0 | 15 | 43 | 28 |
| NaOH 0.1N | RT | 1.20min | 12.9 | 20 | 67 | 47 |

| | | | | | | |
|---|---|---|---|---|---|---|
| * *Average of 3 replicates* | | | | | | |

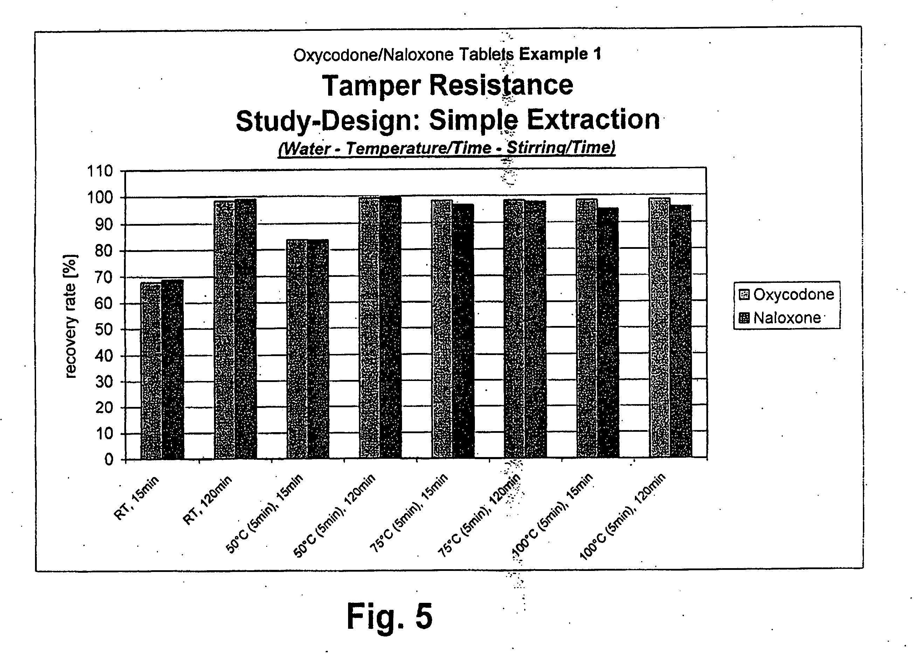

The results are also presented in Figures 5 to 7.

### Test results "simple extraction" using Example 2

| **Solvent** | **Temperature/ Time** | **Stirring/Time** | **PH (Test - solution)** | **% recovery Oxycodone*** | **% recovery Naloxone*** | **Δ % points** |
|---|---|---|---|---|---|---|
| D-Water | RT | 15min | 6.7 | 82 | 83 | 1 |
| D-Water | RT | 120min | 6.7 | 96 | 96 | 0 |
| D-Water | 50°C/5min | 15min | 6.7 | 90 | 90 | 0 |
| D-Water | 50*C/5min | 120min | 6.6 | 98 | 98 | 0 |
| D-Water | 75°C/5min | 15min | 6.9 | 97 | 95 | 2 |
| D-Water | 75°C/5min | 120min | 6.9 | 99 | 97 | 2 |
| D-Water | 100°C/5min | 15min | 7.0 | 98 | 95 | 3 |
| D-Water | 100°/5min | 120min | 7.1 | 98 | 95 | 3 |
| HCl 2N | RT | 15min | not measurable | 65 | 65 | 0 |
| HCl 2N | RT | 120min | not measurable | 98 | 99 | 1 |
| Ethanol 40% | RT | 15min | 6.6 | 79 | 80 | 1 |
| Ethanol 40% | RT | 120min | 6.6 | 97 | 98 | 1 |
| CH₃COOH 2N | RT | 15min | 2.1 | 84 | 84 | 0 |
| CH₃COOH 2N | RT | 120min | 2.1 | 99 | 99 | 0 |
| NaOH 2N | RT | 15min | 13.8 | 4 | 53 | 49 |
| NaOH 2N | RT | 120min | 13.8 | 4 | 63 | 59 |
| NaOH 1N | RT | 15min | 13.7 | 6 | 60 | 54 |
| NaOH 1N | RT | 120min | 13.7 | 6 | 85 | 79 |
| NaOH 0.5N | RT | 15min | 13.4 | 6 | 54 | 48 |
| NaOH 0.5N | RT | 120min | 13.4 | 6 | 83 | 77 |

| **Solvent** | **Temperature/ Time** | **Stirring/Time** | **PH (Test - solution)** | **% recovery Oxycodone*** | **% recovery Naloxone*** | **Δ % points** |
|---|---|---|---|---|---|---|
| NaOH 0.1 N | RT | 15min | 12.8 | 9 | 46 | 87 |
| NaOH 0.1N | RT | 120min | 12.7 | 10 | 76 | 66 |

| | | | | | | |
|---|---|---|---|---|---|---|
| ** Average of 3 replicates* | | | | | | |

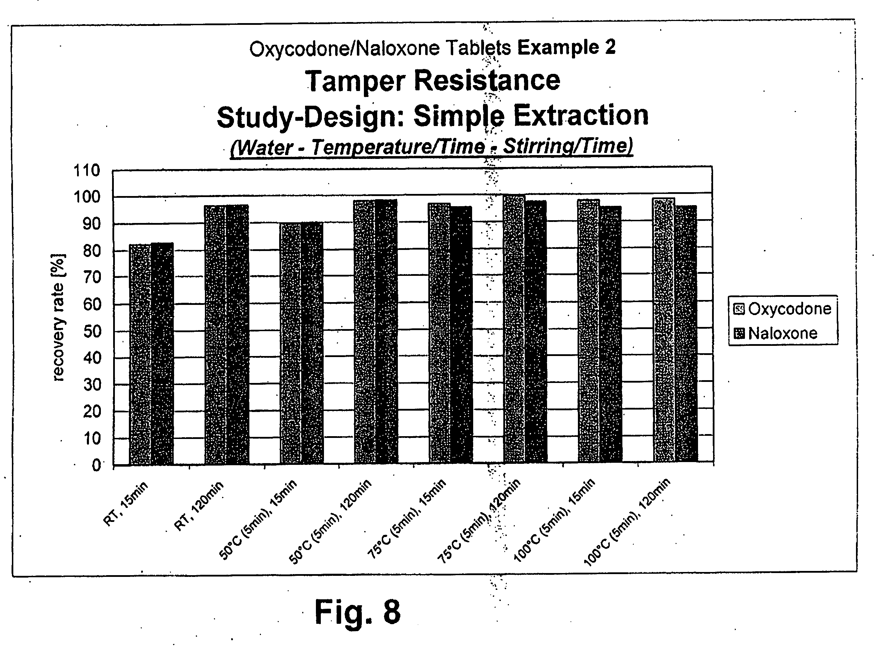

The results are also presented in Figures 8 to 10.

### Test results "additional test"

| **Intact Dosage Form** | **PH (Test-Solution)** | **% recovery Oxycodone*** | **% recovery Naloxone*** | **Δ % points** |
|---|---|---|---|---|
| Example 1 | 6.8 | 99 | 90 | 9 |
| Example 2 | 6.9 | 96 | 94 | 4 |

| | | | | |
|---|---|---|---|---|
| * *Average of 3 replicates* | | | | |

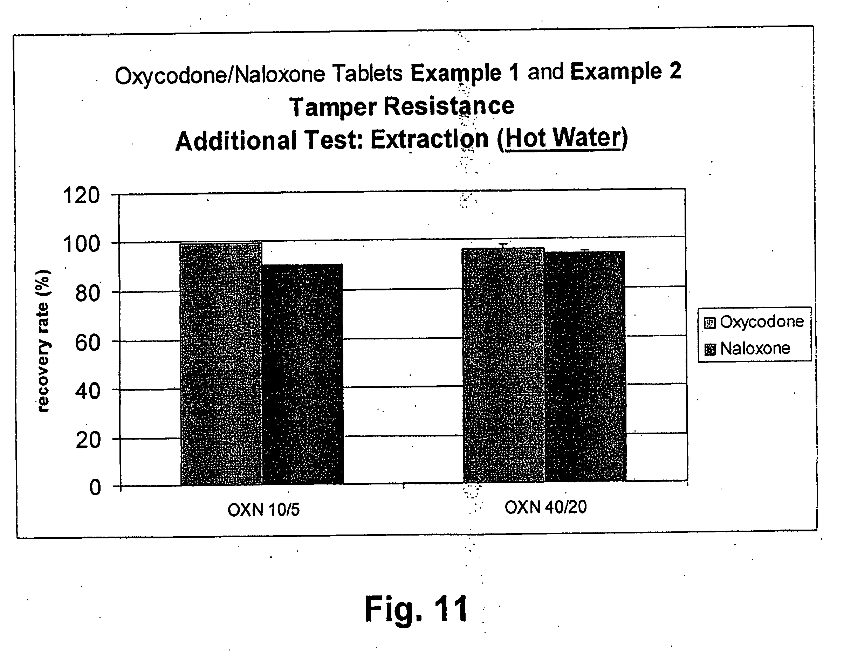

The results are also presented in Figure 11.

The results of all experiments confirm that typical street abuse i.e. separation of the oxycodone from the naloxone from the oxycodone/naloxone tablets is not possible. The difference in the relative amount of oxycodone and naloxone extracted by the test based on the amount present in the extracted tablets (Δ % points) is small in cases where a larger relative amount of oxycodone is extracted.

It is not possible to separate the components oxycodone and naloxone from each other by simple extraction and/or different crushing methods.

The recovery rate of both substances is comparable in all experiments, except simple extraction in basic media. In these experiments, it can be observed, that the concentration of extracted oxycodone is significantly lower than naloxone. After filtration, the remaining mass isn't usable for any conventional abuse activities. It also contains the tablet matrix and oxycodone is soaked with strong caustic. A purification procedure would probably not be practicable on the street, because this would depend on the ability to carry out an advanced extraction.

## Claims

1. Use of an amount of an opioid antagonist in an amount at least sufficient to substantially antagonize a therapeutic amount of opioid agonist, when both, the opioid agonist and the opioid antagonist, are administered intravenous at the same time, in the form of a controlled release dosage form comprising a homogeneous controlled release matrix formulation comprising
a hydrophobic material, including
at least one hydrophobic polymer, wherein the hydrophobic polymer is ethyl cellulose, and
at least one fatty alcohol or fatty acid selected from C₁₂ to C₃₆ aliphatic alcohols or acids,
and said therapeutic amount of an opioid agonist and said sufficient amount of opioid antagonist,
to prevent the formation of an extract of said controlled release matrix formulation comprising the opioid agonist by a one step extraction procedure comprising the steps of:
a) crushing the formulation of one dosage form using a pill crusher or a tablet mortar, or using two spoons, wherein the crushing is performed at least 4 times using the spoons,
b) extracting the crushed formulation of one dosage form on a spoon using 2 ml boiling tap water as extracting agent and a cigarette lighter as heating means for a time period that is necessary to boil the water, and
c) filtering the solution using cotton,
wherein the opioid antagonist is present in said extract in a weight percent amount, based on the total amount of opioid antagonist in the dosage form, that is more than 20 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

2. Use according to claim 1 to prevent the formation of an extract, wherein the opioid antagonist is present in said extract in a weight percent amount, based on the total amount of opioid antagonist in the dosage form, that is more than 15 %-points, preferably more than 12 %-points less than the weight percent amount of opioid agonist present in the extract, based on the total amount of opioid agonist in the dosage form.

3. Use of an amount of an opioid antagonist in an amount at least sufficient to substantially antagonize a therapeutic amount of opioid agonist when both, the opioid agonist and the opioid antagonist, are administered intravenous at the same time, in the form of a controlled release dosage form comprising a homogeneous controlled release matrix formulation comprising
a hydrophobic material, including
at least one hydrophobic polymer, wherein the hydrophobic polymer is ethyl cellulose, and
at least one fatty alcohol or fatty acid selected from C₁₂ to C₃₆ aliphatic alcohols or acids,
and said therapeutic amount of an opioid agonist and said sufficient amount of opioid antagonist,
to prevent the formation of an extract of said controlled release matrix formulation comprising the opioid agonist by a one step extraction procedure comprising the steps of:
a) crushing the formulation of one dosage form using a pill crusher or a tablet mortar, or using two spoons, wherein the crushing is performed at least 4 times using the spoons
b) extracting said crushed formulation on a spoon using 2 ml boiling deionized water as extracting agent and a cigarette lighter as heating means for a time period that is necessary to boil the water, and
c) filtering the solution using cotton,
wherein the opioid antagonist is present in said extract in a weight percent amount, based on the total amount of opioid antagonist in the dosage form, that is more than 15 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

4. Use according to claim 3 to prevent the formation of an extract, wherein the opioid antagonist is present in said extract in a weight percent amount, based on the total amount of opioid antagonist in the dosage form, that is more than 10 %-points, preferably more than 7 %-points less than the weight percent amount of opioid agonist present in the extract, based on the total amount of opioid agonist in the dosage form.

5. Use of an amount of an opioid antagonist in an amount at least sufficient to substantially antagonize a therapeutic amount of opioid agonist, when both, the opioid agonist and the opioid antagonist, are administered intravenous at the same time, in the form of a controlled release dosage form comprising a homogeneous controlled release matrix formulation comprising
a hydrophobic material, including
at least one hydrophobic polymer, wherein the hydrophobic polymer is ethyl cellulose, and
at least one fatty alcohol or fatty acid selected from C₁₂ to C₃₆ aliphatic alcohols or acids,
and said therapeutic amount of an opioid agonist and said sufficient amount of opioid antagonist,
to prevent the formation of an extract of said controlled release matrix formulation comprising the opioid agonist by a one step extraction procedure comprising the steps of:
a) crushing the formulation of 10 dosage forms using a pill crusher
b) extracting said crushed formulation in a glass vial using 100 ml of extraction solvent selected from the group of deionized water, hydrochloric acid (2N), acetic acid (2N), sodium hydroxide solution (0. 1N, 0.5N, 1N or 2 N) and ethanol (40%), and shaking for at least 15 minutes at least at room temperature,
wherein the opioid antagonist is present in said extract in a weight percent amount, based on the total amount of opioid antagonist in the dosage form, that is more than 10 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

6. Use according to claim 5 to prevent the formation of an extract, wherein the opioid antagonist is present in said extract in a weight percent amount, based on the total amount of opioid antagonist in the dosage form, that is more than 5 %-points or more than 3 %-points less than the weight percent amount of opioid agonist present in the extract, based on the total amount of opioid agonist in the dosage form.

7. Use according to claims 5 or 6 to prevent the formation of an extract, wherein shaking is performed for 120 minutes.

8. Use according to any one of claims 5 to 7 to prevent the formation of an extract, wherein deionized water is used as extraction solvent and during extraction the deionized water is heated to 50°C, preferably 75°C and most preferred 100°C for 5 minutes.

9. Use of an amount of an opioid antagonist in an amount at least sufficient to substantially antagonize a therapeutic amount of opioid agonist, when both, the opioid agonist and the opioid antagonist, are administered intravenous at the same time, in the form of a controlled release dosage form comprising a homogeneous controlled release matrix formulation comprising
a hydrophobic material, including
at least one hydrophobic polymer, wherein the hydrophobic polymer is ethyl cellulose, and
at least one fatty alcohol or fatty acid selected from C₁₂ to C₃₆ aliphatic alcohols or acids,
and said therapeutic amount of an opioid agonist and said sufficient amount of opioid antagonist,
to prevent the formation of an extract of said controlled release matrix formulation comprising the opioid agonist by a one step extraction procedure comprising the steps of:
a) heating deionized water to 70°C
b) adding intact formulation of one dosage form and stirring for 15 minutes
c) separating the extract
wherein the opioid antagonist is present in said extract in a weight percent amount, based on the total amount of opioid antagonist in the dosage form, that is more than 15 %-points less than the weight percent amount of opioid agonist present in the extract, based on the total amount of opioid agonist in the dosage form, and wherein the opioid agonist is oxycodone hydrochloride and the opioid antagonist is naloxone hydrochloride which are present in the dosage form in an amount ratio of 2:1.

10. Use according to claim 9 to prevent the formation of an extract, wherein the opioid antagonist is present in said extract in a weight percent amount, based on the total amount of opioid antagonist in the dosage form, that is more than 10 %-points less than the weight percent amount of opioid agonist present in the extract, based on the total amount of opioid agonist in the dosage form.

11. Use according to any one of the preceding claims wherein the formulation is prepared by a melt extrusion step to form a homogenous matrix.

12. Use according to any one of the preceding claims, wherein the amount of ethyl cellulose is less than 20 % (by wt), preferably less than 15 % (by wt), most preferred less than 10 % (by wt) but more than 5 % (by wt) of the matrix formulation.

13. Use according to any one of the preceding claims, wherein the fatty alcohol or fatty acid is selected from stearyl alcohol, cetyl alcohol, cetostearyl alcohol, stearic acid, palmitic acid and mixtures thereof.

14. Use according to claim 13, wherein the amount of C₁₂ to C₃₆ aliphatic alcohol or acid is at least 5 %, more preferred at least 10 % (by wt), more preferred at least 15 % (by wt) and most preferred 20 % to 25 % (by wt) of the matrix formulation.

15. Use according to any one of the preceding claims wherein the amount of ethyl cellulose is less than 10 % (by wt) of the matrix formulation further comprising stearyl alcohol in an amount of between 20% and 25 % (by wt) and oxycodone hydrochloride and naloxone hydrochloride in an amount ratio of 2:1.

## Patentansprüche

1. Verwendung einer Menge eines Opioid-Antagonisten in einer solchen Menge, die mindestens ausreichend ist, um eine therapeutische Menge eines Opioid-Agonisten im Wesentlichen zu antagonisieren, wenn beide, der Opioid-Agonist und der Opioid-Antagonist, zur selben Zeit intravenös verabreicht werden, in der Form einer kontrolliert-freisetzenden Dosierungsform umfassend eine homogene kontrolliertfreisetzende Matrixformulierung umfassend
ein hydrophobes Material, einschließend
mindestens ein hydrophobes Polymer, wobei das hydrophobe Polymer Ethylcellulose ist, und
mindestens einen Fettalkohol oder eine Fettsäure ausgewählt aus C₁₂ bis C₃₆ aliphatischen Alkoholen oder Säuren,
und die besagte therapeutische Menge eines Opioid-Agonisten und die besagte ausreichende Menge des Opioid-Antagonisten,
um die Bildung eines Extrakts der besagten kontrolliert-freisetzenden Matrixformulierung, der den Opioid-Agonisten umfasst, zu verhindern, und zwar einen Extrakt aus einem Einschritt-Extraktionsverfahren umfassend die Schritte aus:
a) Zerdrücken der Formulierung einer Dosierungsform unter der Verwendung eines Pillen-Crushers oder eines Tablettenmörsers, oder unter der Verwendung von zwei Löffeln, wobei das Zerdrücken unter der Verwendung der Löffel mindestens 4 Mal durchgeführt wird,
b) Extraktion der zerdrückten Formulierung einer Dosierungsform auf einem Löffel unter der Verwendung von 2 ml kochendem Leitungswasser als Extraktionsagens und einem Zigarettenfeuerzeug als Hitzequelle für einen Zeitraum, der notwendig ist, um das Wasser zum Kochen zu bringen, und
c) Filtern der Lösung unter der Verwendung von Watte,
wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 20 %-Punkte geringer ist als die Gewichtsprozentmenge des Opioid-Agonisten, der im Extrakt vorhanden ist, basierend auf der Gesamtmenge des pioid-Agonisten in der Dosierungsform, und wobei der Opioid-Agonist Oxycodonhydrochlorid ist und der Opioid-Antagonist Naloxonhydrochlorid ist, die in der Dosierungsform in einem Mengenverhältnis von 2:1 vorhanden sind.

2. Verwendung gemäß Anspruch 1 um die Bildung eines Extrakts zu verhindern, wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 15 %-Punkte, bevorzugt mehr als 12 %-Punkte geringer ist als die Gewichtsprozentmenge des Opioid-Agonisten, der im Extrakt vorhanden ist, basierend auf der Gesamtmenge des Opioid-Agonisten in der Dosierungsform.

3. Verwendung einer Menge eines Opioid-Antagonisten in einer solchen Menge, die mindestens ausreichend ist, um eine therapeutische Menge eines Opioid-Agonisten im Wesentlichen zu antagonisieren, wenn beide, der Opioid-Agonist und der pioid-Antagonist, zur selben Zeit intravenös verabreicht werden, in der Form einer kontrolliert-freisetzenden Dosierungsform umfassend eine homogene kontrolliertfreisetzende Matrixformulierung umfassend
ein hydrophobes Material, einschließend
mindestens ein hydrophobes Polymer, wobei das hydrophobe Polymer Ethylcellulose ist, und
mindestens einen Fettalkohol oder eine Fettsäure ausgewählt aus C₁₂ bis C₃₆ aliphatischen Alkoholen oder Säuren,
und die besagte therapeutische Menge eines Opioid-Agonisten und die besagte ausreichende Menge des Opioid-Antagonisten,
um die Bildung eines Extrakts der besagten kontrolliert-freisetzenden Matrixformulierung, der den Opioid-Agonisten umfasst, zu verhindern, und zwar einen Extrakt aus einem Einschritt-Extraktions verfahren umfassend die Schritte aus:
a) Zerdrücken der Formulierung einer Dosierungsform unter der Verwendung eines Pillen-Crushers oder eines Tablettenmörsers, oder unter der Verwendung von zwei Löffeln, wobei das Zerdrücken unter der Verwendung der Löffel mindestens 4 Mal durchgeführt wird,
b) Extraktion der besagten zerdrückten Formulierung auf einem Löffel unter der Verwendung von 2 ml kochendem entionisierten Wasser als Extraktionsagens und einem Zigarettenfeuerzeug als Hitzequelle für einen Zeitraum, der notwendig ist, um das Wasser zum Kochen zu bringen, und
c) Filtern der Lösung unter der Verwendung von Watte,
wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 15 %-Punkte geringer ist als die Gewichtsprozentmenge des Opioid-Agonisten, der im Extrakt vorhanden ist, basierend auf der Gesamtmenge des Opioid-Agonisten in der Dosierungsform, und wobei der Opioid-Agonist Oxycodonhydrochlorid ist und der Opioid-Antagonist Naloxonhydrochlorid ist, die in der Dosierungsform in einem Mengenverhältnis von 2:1 vorhanden sind.

4. Verwendung gemäß Anspruch 3 um die Bildung eines Extrakts zu verhindern, wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 10 %-Punkte, bevorzugt mehr als 7 %-Punkte geringer ist als die Gewichtsprozentmenge des Opioid-Agonisten, der im Extrakt vorhanden ist, basierend auf der Gesamtmenge des Opioid-Agonisten in der Dosierungsform.

5. Verwendung einer Menge eines Opioid-Antagonisten in einer solchen Menge, die mindestens ausreichend ist, um eine therapeutische Menge eines Opioid-Agonisten im Wesentlichen zu antagonisieren, wenn beide, der Opioid-Agonist und der Opioid-Antagonist, zur selben Zeit intravenös verabreicht werden, in der Form einer kontrolliert-freisetzenden Dosierungsform umfassend eine homogene kontrolliertfreisetzende Matrixformulierung umfassend
ein hydrophobes Material, einschließend
mindestens ein hydrophobes Polymer, wobei das hydrophobe Polymer Ethylcellulose ist, und
mindestens einen Fettalkohol oder eine Fettsäure ausgewählt aus C₁₂ bis C₃₆ aliphatischen Alkoholen oder Säuren,
und die besagte therapeutische Menge eines Opioid-Agonisten und die besagte ausreichende Menge des Opioid-Antagonisten,
um die Bildung eines Extrakts der besagten kontrolliert-freisetzenden Matrixformulierung, der den Opioid-Agonisten umfasst, zu verhindern, und zwar einen Extrakt aus einem Einschritt-Extraktionsverfahren umfassend die Schritte aus:
a) Zerdrücken der Formulierung von 10 Dosierungsformen unter der Verwendung eines Pillen-Crushers,
b) Extraktion der besagten zerdrückten Formulierung in einem Glasgefäß unter der Verwendung von 100 ml eines Extraktionslösungsmittels ausgewählt aus der Gruppe aus entionisiertem Wasser, hydrochlorischer Säure (2N), Essigsäure (2N), Natriumhydroxid-Lösung (0.1N, 0.5N, 1N oder 2 N) und Ethanol (40%), und Schütteln für mindestens 15 Minuten bei mindestens Raumtemperatur,
wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 10 %-punkte geringer ist als die Gewichtsprozentmenge des Opioid-Agonisten, der im Extrakt vorhanden ist, basierend auf der Gesamtmenge des Opioid-Agonisten in der Dosierungsform, und wobei der Opioid-Agonist Oxycodonhydrochlorid ist und der Opioid-Antagonist Naloxonhydrochlorid ist, die in der Dosierungsform in einem Mengenverhältnis von 2:1 vorhanden sind.

6. Verwendung gemäß Anspruch 5 um die Bildung eines Extrakts zu verhindern, wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 5 %-Punkte oder mehr als 3 %-Punkte geringer ist als die Gewichtsprozentmenge des Opioid-Agonisten, der im Extrakt vorhanden ist, basierend auf der Gesamtmenge des Opioid-Agonisten in der Dosierungsform.

7. Verwendung gemäß den Anspüchen 5 oder 6 um die Bildung eines Extrakts zu verhindern, wobei das Schütteln für 120 Minuten ausgeführt wird.

8. Verwendung gemäß eines jeden der Ansprüche 5 bis 7 um die Bildung eines Extrakts zu verhindern, wobei entionisiertes Wassser als Extraktions-Lösungsmittel verwendet wird und das entionisierte Wasser während der Extraktion auf 50°C, bevorzugt auf 70°C und am meisten bevorzugt auf 100°C für 5 Minuten erhitzt wird.

9. Verwendung einer Menge eines Opioid-Antagonisten in einer solchen Menge, die mindestens ausreichend ist, um eine therapeutische Menge eines Opioid-Agonisten im Wesentlichen zu antagonisieren, wenn beide, der Opioid-Agonist und der Opioid-Antagonist, zur selben Zeit intravenös verabreicht werden, in der Form einer kontrolliert-freisetzenden Dosierungsform umfassend eine homogene kontrolliertfreisetzende Matrixformunlierung umfassend
ein hydrophobes Material, einschließend
mindestens ein hydrophobes Polymer, wobei das hydrophobe Polymer Ethylcellulose ist, und
mindestens einen Fettalkohol oder eine Fettsäure ausgewählt aus C₁₂ bis C₃₆ aliphatischen Alkoholen oder Säuren,
und die besagte therapeutische Menge eines Opioid-Agonisten und die besagte ausreichende Menge des Opioid-Antagonisten,
um die Bildung eines Extrakts der besagten kontrolliert-freisetzenden Matrixformulierung, der den Opioid-Agonisten umfasst, zu verhindern, und zwar einen Extrakt aus einem Einschritt-Extraktionsverfahren umfassend die Schritte aus:
a) Aufheizen von entionisiertem Wasser auf 70°C,
b) Zugabe der intakten Formulierung einer Dosierungsform und Rühren für 15 Minuten,
c) Auftrennen des Extrakts,
wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 15 %-Punkte geringer ist als die Gewichtsprozentmenge des Opioid-Agonisten, der im Extrakt vorhanden ist, basierend auf der Gesamtmenge des Opioid-Agonisten in der Dosierungsform, und wobei der Opioid-Agonist Oxycodonhydrochlorid ist und der Opioid-Antagonist Naloxonhydrochlorid ist, die in der Dosierungsform in einem Mengenverhältnis von 2:1 vorhanden sind.

10. Verwendung gemäß Anspruch 9 um die Bildung eines Extrakts zu verhindern, wobei der Opioid-Antagonist in besagtem Extrakt in einer Gewichtsprozentmenge, basierend auf der Gesamtmenge des Opioid-Antagonisten in der Dosierungsform, vorhanden ist, die mehr als 10 %-Punkte geringer ist als die Gewichtsprozentmenge des Opioid-Agonisten, der im Extrakt vorhanden ist, basierend auf der Gesamtmenge des Opioid-Agonisten in der Dosierungsform.

11. Verwendung gemäß eines jeden der vorangegangenen Ansprüche, wobei die Formulierung durch einen Schmelzextrusionsschritt hergestellt ist, um eine homogene Matrix zu bilden.

12. Verwendung gemäß eines jeden der vorangegangenen Ansprüche, wobei die Menge der Ethylcellulose weniger als 20 % (Gewichtsprozent), bevorzugter weniger als 15 % (Gewichtsprozent), am meisten bevorzugt weniger als 10 % (Gewichtsprozent) aber mehr als 5 % (Gewichtsprozent) der Matrixformulierung beträgt.

13. Verwendung gemäß eines jeden der vorangegangenen Ansprüche, wobei der Fettalkohol oder die Fettsäure ausgewählt ist aus Sterylalkohol, Cetylalkohol, Cetostearylalkohol, Stearinsäure, Palmitinsäure und Mischungen davon.

14. Verwendung gemäß Anspruch 13, wobei die Menge des C₁₂ bis C₃₆ aliphatischen Alkohols oder der Säure mindestens 5 %, bevorzugter mindestens 10 % (Gewichtsprozent), bevorzugter mindestens 15 % (Gewichtsprozent) und am meisten bevorzugt 20 % bis 25 % (Gewichtsprozent) der Matrixformulierung beträgt.

15. Verwendung gemäß eines jeden der vorangegangenen Ansprüche, wobei die Menge der Ethylcellulose weniger als 10% (Gewichtsprozent) der Matrixformulierung beträgt, wobei die Formulierung darüberhinaus Stearylalkohol in einer Menge von zwischen 20 % und 25 % (Gewichtsprozent) und Oxycodonhydrochlorid und Naloxonhydrochlorid in einem Mengenverhältnis von 2:1 umfasst.

## Revendications

1. Utilisation d'une quantité d'un antagoniste d'opioïde en une quantité au moins suffisante pour antagoniser substantiellement une quantité thérapeutique d'un agoniste d'opioïde, lorsque les deux, l'agoniste d'opioïde et l'antagoniste d'opioïde, sont administrés par voie intraveineuse en même temps, sous forme d'une forme posologique à libération contrôlée comprenant une formulation de matrice homogène à libération contrôlée comprenant
une matière hydrophobe, comprenant
au moins un polymère hydrophile, ledit polymère hydrophile étant l'éthylcellulose, et
au moins un alcool gras ou acide gras choisi parmi des alcools ou acides aliphatiques en C₁₂ à C₃₆,
et ladite quantité thérapeutique d'un agoniste d'opioïde et ladite quantité suffisante d'antagoniste d'opioïde,
pour empêcher la formation d'un extrait de ladite formulation de matrice à libération contrôlée comprenant l'agoniste d'opioïde par un mode opératoire d'extraction en une étape comprenant les étapes consistant :
a) à écraser la formulation d'une forme posologique en utilisant un broyeur de pilules ou un mortier pour comprimés, ou bien en utilisant deux cuillères, ledit écrasement étant effectué au moins 4 fois en utilisant les cuillères,
b) à extraire la formulation écrasée d'une forme posologique sur une cuillère en utilisant 2 ml d'eau du robinet bouillante comme agent d'extraction et un briquet comme moyen de chauffage pendant une période de temps qui est nécessaire pour faire bouillir l'eau, et
c) à filtrer la solution en utilisant du coton,
dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 20 points de % à la quantité en pourcentage en poids d'agoniste d'opioïde présente dans l'extrait, sur la base de la quantité totale d'agoniste d'opioïde dans la forme posologique, et dans laquelle l'agoniste d'opioïde est le chlorhydrate d'oxycodone et l'antagoniste d'opioïde est le chlorhydrate de naloxone qui sont présents dans la forme posologique en un rapport des quantités de 2:1.

2. Utilisation suivant la revendication 1 pour empêcher la formation d'un extrait, dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 15 points de %, de préférence de plus de 12 points de %, à la quantité en pourcentage en poids d'agoniste d'opioïde présente dans l'extrait, sur la base de la quantité totale d'agoniste d'opioïde dans la forme posologique.

3. Utilisation d'une quantité d'un antagoniste d'opioïde correspondant à une quantité au moins suffisante pour antagoniser substantiellement une quantité thérapeutique d'un agoniste d'opioïde lorsque les deux, l'agoniste d'opioïde et l'antagoniste d'opioïde, sont administrés par voie intraveineuse en même temps, sous forme d'une forme posologique à libération contrôlée comprenant une formulation de matrice homogène à libération contrôlée comprenant
une matière hydrophobe, comprenant
au moins un polymère hydrophobe, ledit polymère hydrophobe étant l'éthylcellulose, et
au moins un alcool gras ou acide gras choisi parmi des alcools ou acides aliphatiques en C₁₂ à C₃₆,
et ladite quantité thérapeutique d'un agoniste d'opioïde et ladite quantité suffisante d'antagoniste d'opioïde,
pour empêcher la formation d'un extrait de ladite formulation de matrice à libération contrôlée comprenant l'agoniste d'opioïde par un mode opératoire d'extraction en une étape comprenant les étapes consistant :
a) à écraser la formulation d'une forme posologique en utilisant un broyeur de pilules ou un mortier pour comprimés, ou bien en utilisant deux cuillères, l'écrasement étant effectué au moins 4 fois en utilisant les cuillères,
b) à soumettre à une extraction ladite formulation écrasée sur une cuillère en utilisant 2 ml d'eau désionisée bouillante comme agent d'extraction et un briquet comme moyen de chauffage pendant une période de temps qui est nécessaire pour faire bouillir l'eau, et
c) à filtrer la solution en utilisant du coton,
dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 15 points de % à la quantité en pourcentage en poids d'agoniste d'opioïde présente dans l'extrait, sur la base de la quantité totale d'agoniste d'opioïde dans la forme posologique, et dans laquelle l'agoniste d'opioïde est le chlorhydrate d'oxycodone et l'antagoniste d'opioïde est le chlorhydrate de naloxone qui sont présents dans la forme posologique en un rapport des quantités de 2:1.

4. Utilisation suivant la revendication 3 pour empêcher la formation d'un extrait, dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 10 points de %, de préférence de plus de 7 points de %, à la quantité en pourcentage en poids d'agoniste d'opioïde présente dans l'extrait, 1 sur la base de la quantité totale d'agoniste d'opioïde dans la forme posologique.

5. Utilisation d'une quantité d'un antagoniste d'opioïde correspondant à une quantité au moins suffisante pour antagoniser substantiellement une quantité thérapeutique d'un agoniste d'opioïde lorsque les deux, l'agoniste d'opioïde et l'antagoniste d'opioïde, sont administrés par voie intraveineuse en même temps, sous forme d'une forme posologique à libération contrôlée comprenant une formulation de matrice homogène à libération contrôlée comprenant
une matière hydrophobe, comprenant
au moins un polymère hydrophobe, ledit polymère hydrophobe étant l'éthylcellulose, et
au moins un alcool gras ou acide gras choisi parmi des alcools ou acides aliphatiques en C₁₂ à C₃₆,
et ladite quantité thérapeutique d'un agoniste d'opioïde et ladite quantité suffisante d'antagoniste d'opioïde,
pour empêcher la formation d'un extrait de ladite formulation de matrice à libération contrôlée comprenant l'agoniste d'opioïde par un mode opératoire d'extraction en une étape comprenant les étapes consistant :
a) à écraser la formulation de 10 formes posologiques en utilisant un broyeur de pilules,
b) à soumettre à une extraction ladite formulation écrasée dans un flacon en verre en utilisant 100 ml d'un solvant d'extraction choisi dans le groupe comprenant l'eau désionisée, l'acide chlorhydrique (2N), l'acide acétique (2N), une solution d'hydroxyde de sodium (0,1 N, 0,5 N, 1 N ou 2 N) et l'éthanol (40 %), et effectuer une agitation par secousse pendant au moins 15 minutes au moins à température ambiante,
dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 10 points de % à la quantité en pourcentage en poids d'agoniste d'opioïde présente dans l'extrait, sur la base de la quantité totale d'agoniste d'opioïde dans la forme posologique, et dans laquelle l'agoniste d'opioïde est le chlorhydrate d'oxycodone et l'antagoniste d'opioïde est le chlorhydrate de naloxone qui sont présents dans la forme posologique en un rapport des quantités de 2:1.

6. Utilisation suivant la revendication 5 pour empêcher la formation d'un extrait, dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 5 points de % ou de plus de 3 points de %, à la quantité en pourcentage en poids d'agoniste d'opioïde présente dans l'extrait, sur la base de la quantité totale d'agoniste d'opioïde dans la forme posologique.

7. Utilisation suivant la revendication 5 ou 6 pour empêcher la formation d'un extrait, dans laquelle l'agitation par secousse est effectuée pendant 120 minutes.

8. Utilisation suivant l'une quelconque des revendications 5 à 7 pour empêcher la formation d'un extrait, dans laquelle de l'eau désionisée est utilisée comme solvant d'extraction et, au cours de l'extraction, l'eau désionisée est chauffée à 50°C, avantageusement à 75°C, de préférence à 100°C pendant 5 minutes.

9. Utilisation d'une quantité d'un antagoniste d'opioïde correspondant à une quantité au moins suffisante pour antagoniser substantiellement une quantité thérapeutique d'un agoniste d'opioïde lorsque les deux, l'agoniste d'opioïde et l'antagoniste d'opioïde, sont administrés par voie intraveineuse en même temps, sous forme d'une forme posologique à libération contrôlée comprenant une formulation de matrice homogène à libération contrôlée comprenant
une matière hydrophobe, comprenant
au moins un polymère hydrophobe, ledit polymère hydrophobe étant l'éthylcellulose, et
au moins un alcool gras ou acide gras choisi parmi des alcools ou acides aliphatiques en C₁₂ à C₃₆,
et ladite quantité thérapeutique d'un agoniste d'opioïde et ladite quantité suffisante d'antagoniste d'opioïde,
pour empêcher la formation d'un extrait de ladite formulation de matrice à libération contrôlée comprenant l'agoniste d'opioïde par un mode opératoire d'extraction en une étape comprenant les étapes consistant :
a) à chauffer l'eau désionisée à 70°C
b) ajouter la formulation intacte d'une forme posologique et à effectuer une agitation pendant 15 minutes
c) à séparer l'extrait
dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 15 points de % à la quantité en pourcentage en poids d'agoniste d'opioïde présente dans l'extrait, sur la base de la quantité totale d'agoniste d'opioïde dans la forme posologique, et dans laquelle l'agoniste d'opioïde est le chlorhydrate d'oxycodone et l'antagoniste d'opioïde est le chlorhydrate de naloxone qui sont présents dans la forme posologique en un rapport des quantités de 2:1.

10. Utilisation suivant la revendication 9 pour empêcher la formation d'un extrait, dans laquelle l'antagoniste d'opioïde est présent dans ledit extrait en une quantité en pourcentage en poids, sur la base de la quantité totale d'antagoniste d'opioïde dans la forme posologique, qui est inférieure de plus de 10 points de % à la quantité en pourcentage en poids d'agoniste d'opioïde présente dans l'extrait, sur la base de la quantité totale d'agoniste d'opioïde dans la forme posologique.

11. Utilisation suivant l'une quelconque des revendications précédentes, dans laquelle la formulation est préparée par une étape d'extrusion en masse fondue pour former une matrice homogène.

12. Utilisation suivant l'une quelconque des revendications précédentes, dans laquelle la quantité d'éthylcellulose est inférieure à 20 % (en poids), avantageusement inférieure à 15 % (en poids), de préférence inférieure à 10 % (en poids) mais supérieure à 5 % (en poids) de la formulation de matrice.

13. Utilisation suivant l'une quelconque des revendications précédentes, dans laquelle l'alcool gras ou l'acide gras est choisi entre l'alcool stéarylique, l'alcool cétylique, l'alcool cétostéarylique, l'acide stéarique, l'acide palmitique et leurs mélanges.

14. Utilisation suivant la revendication 13, dans laquelle la quantité d'alcool ou d'acide aliphatique en C₁₂ à C₃₆ est égale à au moins 5 %, plus avantageusement égale à au moins 10 % (en poids), mieux encore également à 15 % (en poids), de préférence de 20 % à 25 % (en poids) de la formulation de matrice.

15. Utilisation suivant l'une quelconque des revendications précédentes, dans laquelle la quantité d'éthylcellulose est inférieure à 10 % (en poids) de la formulation de matrice comprenant en outre de l'alcool stéarylique en une quantité comprise entre 20 % et 25 % (en poids) et du chlorhydrate d'oxycodone et du chlorhydrate d'oxycodone et du chlorhydrate de naloxone en un rapport des quantités de 2:1.
